(19) Europäisches Patentamt
European Patent Office
Office européen des brevets

(11) Veröffentlichungsnummer: **0 232 779**
**B1**

(12) # EUROPÄISCHE PATENTSCHRIFT

(45) Veröffentlichungstag der Patentschrift: **12.10.88**

(21) Anmeldenummer: **87100993.2**

(22) Anmeldetag: **24.01.87**

(51) Int. Cl.⁴: **C 07 C 39/23**, C 07 C 59/72, C 07 C 69/712, A 61 K 31/16, C 07 C 43/168

(54) **Vinylphenolderivate, ihre Herstellung und Verwendung.**

(30) Priorität: **28.01.86 DE 3602473**

(43) Veröffentlichungstag der Anmeldung: **19.08.87 Patentblatt 87/34**

(45) Bekanntmachung des Hinweises auf die Patenterteilung: **12.10.88 Patentblatt 88/41**

(84) Benannte Vertragsstaaten: **AT BE CH DE ES FR GB GR IT LI NL SE**

(56) Entgegenhaltungen: **EP-A-0 058 370**

(73) Patentinhaber: **BASF Aktiengesellschaft, Carl-Bosch- Strasse 38, D-6700 Ludwigshafen (DE)**

(72) Erfinder: **Wuest, Hans- Heiner, Dr., Unteres Bieth 10, D-6901 Dossenheim (DE)**
Erfinder: **Janssen, Bernd, Dr., Leuschnerstrasse 18 a, D-6700 Ludwigshafen (DE)**
Erfinder: **Frickel, Fritz- Frieder, Dr., Silvanerweg 7, D-6705 Deidesheim (DE)**
Erfinder: **Nuerrenbach, Axel Dr., Koenigsberger Strasse 7, D-6718 Gruenstadt (DE)**

**EP 0 232 779 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Vinylphenolderivate, Verfahren zu deren Herstellung, sowie deren Verwendung bei der Bekämpfung von Krankheiten.

Aus DE-OS-2 854 354 und DE-OS-3 202 118 ist bekannt, daß Vinylbenzoesäurederivate pharmakologische Wirkungen bei der topischen und systemischen Therapie von Neoplasien, Akne, Psoriasis und anderen dermatologischen Affektionen aufweisen. Deren Wirkung ist jedoch nicht immer befriedigend, vor allem wegen unerwünschter Nebenwirkungen.

Es wurde nun gefunden, daß Vinylphenolderivate der Formel I

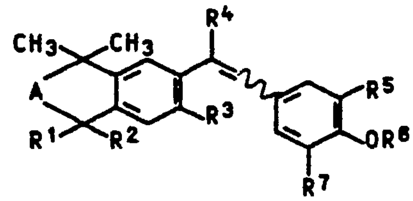

in der

A einen gegebenenfalls mit $C_1$-$C_4$-Alkylgruppen, vorzugsweise Methylgruppen substituierten Methylen- oder Ethylenrest,

$R^1$ und $R^2$ Wasserstoffatome oder Methylgruppen,

$R^3$ ein Wasserstoffatom, eine $C_1$-$C_4$-Alkylgruppe, eine $C_1$-$C_4$-Alkoxygruppe oder ein Halogenatom,

$R^4$ ein Wasserstoffatom, eine $C_1$-$C_4$-Alkylgruppe, eine Cyclopropyl- oder Cyclobutylgruppe,

$R^5$ und $R^7$ je ein Wasserstoffatom oder die Gruppe -$OR^8$, worin $R^8$ für ein Wasserstoffatom, eine $C_1$-$C_4$-Alkylgruppe oder eine $C_1$-$C_4$-Alkanoyl-gruppe steht, und

$R^6$ ein Wasserstoffatom, eine gegebenenfalls mit Carboxyl-, $C_1$-$C_4$-Alkoxy-carbonyl, Hydroxyl-, $C_1$-$C_4$-Alkoxy- und/oder Amino-, Mono- oder Di-$C_1$-$C_4$-Alkylaminogruppen substituierte $C_1$-$C_4$-Alkylgruppe, eine $C_1$-$C_{20}$-Alkanoylgruppe, eine gegebenenfalls wie für die $C_1$-$C_4$-Alkylgruppe beschrieben substituierte Benzoyl- oder eine gegebenenfalls im Arylteil ebenso substituierte Aralkylgruppe darstellen,

sowie deren physiologisch verträgliche Salze ein besseres Wirkungsspektrum mit geringerer Toxizität und höherer therapeutischer Breite besitzen.

$R^6$ und $R^8$ können auch gemeinsam für einen Rest $>C=O$, $>C=S$, -$CH_2CH_2$- oder $>CR^9R^{10}$ stehen mit $R^9$ und $R^{10}$ in der Bedeutung eines Wasserstoffatoms, einer $C_1$-$C_4$-Alkylgruppe oder einer Phenylgruppe.

Als Arylgruppe ist die Phenylgruppe bevorzugt, die mit einer Methyl-, Methoxy- oder Nitrogruppe subtituiert sein kann; als Aralkylgruppe ist die Benzylgruppe bevorzugt, die im Arylteil mit einer Methyl- oder Methoxygruppe oder mit Halogen substituiert sein kann. Substituenten der Benzoylgruppe können beispielsweise die Methyl- oder Methoxygruppe oder Halogen sein. Als Halogenatome sind für $R^3$ vorzugsweise Fluor und Chlor zu nennen.

Typische Beispiele für erfindungsgemäße Verbindungen sind:

1-(4-Hydroxyphenyl)-2-(5,6,7,8-tetrahydro-5,5,8,8-tetramethyl-2-naphthalenyl)propen
1-(4-Methoxyphenyl)-2-(5,6,7,8-tetrahydro-5,5,8,8-tetramethyl-2-naphthalenyl)propen
1-(4-Ethoxyphenyl)-2-(5,6,7,8-tetrahydro-5,5,8,8-tetramethyl-2-naphthalenyl)propen
1-(4-Propyloxyphenyl)-2-(5,6,7,8-tetrahydro-5,5,8,8-tetramethyl-2-naphthalenyl)propen
1-(4-Butoxyphenyl)-2-(5,6,7,8-tetrahydro-5,5,8,8-tetramethyl-2-naphthalenyl)propen
1-(4-Isopropyloxyphenyl)-2-(5,6,7,8-tetrahydro-5,5,8,8-tetramethyl-2-naphthalenyl)propen
1-(4-t-Butyloxyphenyl)-2-(5,6,7,8-tetrahydro-5,5,8,8-tetramethyl-2-naphthalenyl)propen
1-(4-Formyloxyphenyl)-2-(5,6,7,8-tetrahydro-5,5,8,8-tetramethyl-2-naphthalenyl)propen
1-(4-Acetoxyphenyl)-2-(5,6,7,8-tetrahydro-5,5,8,8-tetramethyl-2-naphthalenyl)propen
1-(4-Propionyloxyphenyl)-2-(5,6,7,8-tetrahydro-5,5,8,8-tetramethyl-2-naphthalenyl)propen
1-(4-Palmitoyloxyphenyl)-2-(5,6,7,8-tetrahydro-5,5,8,8-tetramethyl-2-naphthalenyl)propen
1-(4-Stearoyloxyphenyl)-2-(5,6,7,8-tetrahydro-5,5,8,8-tetramethyl-2-naphthalenyl)propen
1-(4-Benzoyloxyphenyl)-2-(5,6,7,8-tetrahydro-5,5,8,8-tetramethyl-2-naphthalenyl)propen
1-[4-(4-Methylbenzoyl)oxyphenyl]-2-(5,6,7,8-tetrahydro-5,5,8,8-tetramethyl-2-naphthalenyl)propen
1-[4-(4-Methoxybenzoyl)oxyphenyl]-2-(5,6,7,8-tetrahydro-5,5,8,8-tetramethyl-2-naphthalenyl)propen
1-[4-(3-Chlorbenzoyl)oxyphenyl]-2-(5,6,7,8-tetrahydro-5,5,8,8-tetramethyl-2-naphthalenyl)propen
1-[4-Benzyloxiphenyl]-2-(5,6,7,8-tetrahydro-5,5,8,8-tetramethyl-2-naphthalenyl)propen
1-[4-(4-Methoxybenzyl)oxyphenyl]-2-(5,6,7,8-tetrahydro-5,5,8,8-tetramethyl-2-naphthalenyl)propen
1-[4-(4-Methylbenzyl)oxyphenyl]-2-(5,6,7,8-tetrahydro-5,5,8,8-tetramethyl-2-naphthalenyl)propen
1-[4-(3-Fluorbenzyl)oxyphenyl]-2-(5,6,7,8-tetrahydro-5,5,8,8-tetramethyl-2-naphthalenyl)propen
1-[4-(2-Hydroxyethyl)oxyphenyl]-2-(5,6,7,8-tetrahydro-5,5,8,8-tetramethyl-2-naphthalenyl)propen
1-[4-(2-Methoxyethyl)oxyphenyl]-2-(5,6,7,8-tetrahydro-5,5,8,8-tetramethyl-2-naphthalenyl)propen
1-[4-(2,3-Dihydroxypropyl)oxyphenyl]-2-(5,6,7,8-tetrahydro-5,5,8,8-tetramethyl-2-naphthalenyl)propen
1-[4-(2-Aminoethyl)oxyphenyl]-2-(5,6,7,8-tetrahydro-5,5,8,8-tetramethyl-2-naphthalenyl)propen

1-[4-(2-N-Methylaminoethyl)oxyphenyl]-2-(5,6,7,8-tetrahydro-5,5,8,8-tetramethyl-2-naphthalenyl)propen
1-[4-(2-N-Ethylaminoethyl)oxyphenyl]-2-(5,6,7,8-tetrahydro-5,5,8,8-tetramethyl-2-naphthalenyl)propen
1-[4-(2-N,N-Dimethylaminoethyl)oxyphenyl]-2-(5,6,7,8-tetrahydro-5,5,8,8-tetramethyl-2-naphthalenyl)propen
1-[4-(2-N,N-Dimethylaminoethyl)oxyphenyl]-2-(5,6,7,8-tetrahydro-5,5,8,8-tetramethyl-2-naphthalenyl)propen
4-[2-(5,6,7,8-Tetrahydro-5,5,8,8-tetramethyl-2-naphthalenyl)-1-propenyl]-phenoxyessigsäuremethylester
4-[2-(5,6,7,8-Tetrahydro-5,5,8,8-tetramethyl-2-naphthalenyl)-1-propenyl]-phenoxyessigsäureethylester
4-[2-(5,6,7,8-Tetrahydro-5,5,8,8-tetramethyl-2-naphthalenyl)-1-propenyl]-phenoxyessigsäure
1-(3,4-Dihydroxyphenyl)-2-(5,6,7,8-tetrahydro-5,5,8,8-tetramethyl-2-naphthalenyl)propen
1-(3-Hydroxy-4-methoxyphenyl)-2-(5,6,7,8-tetrahydro-5,5,8,8-tetrahydro-5,5,8,8-tetramethyl-2-naphthalenyl)propen
1-(4-Hydroxy-3-methoxyphenyl)-2-(5,6,7,8-tetrahydro-5,5,8,8-tetramethyl-2-naphthalenyl)propen
1-(4-Ethoxy-3-hydroxyphenyl)-2-(5,6,7,8-tetrahydro-5,5,8,8-tetramethyl-2-naphthalenyl)propen
1-(3,4-Dimethoxyphenyl)-2-(5,6,7,8-tetrahydro-5,5,8,8-tetramethyl-2-naphthalenyl)propen
5-[1-(2-(5,6,7,8-tetrahydro-5,5,8,8-tetramethyl-2-naphthalenyl)-1-propenyl]-1,3-benzodioxol
2-Methyl-5-[2-(5,6,7,8-tetrahydro-5,5,8,8-tetramethyl-2-naphthalenyl)-1-propenyl]-1,3-benzodioxol
2,2-Dimethyl-5-[2-(5,5,8,8-tetramethyl-2-naphthalenyl)-1-propenyl]-1,3-benzodioxol
2-Phenyl-5-[2-(5,6,7,8-tetrahydro-5,5,8,8-tetramethyl-2-naphthalenyl)-1-propenyl]-1,3-benzodioxol
2-Oxo-5-[2-(5,6,7,8-tetrahydro-5,5,8,8-tetramethyl-2-naphthalenyl)-1-propenyl]-1,3-benzodioxol
2-Thioxo-5-[2-(5,6,7,8-tetrahydro-5,5,8,8-tetramethyl-2-naphthalenyl)-1-propenyl]-1,3-benzodioxol
6-[2-(5,6,7,8-tetrahydro-5,5,8,8-tetramethyl-2-naphthalenyl)-1-propenyl]-1,4-benzodioxan
1-(4-Acetoxy-3-hydroxyphenyl)-2-(5,6,7,8-tetrahydro-5,5,8,8-tetramethyl-2-naphthalenyl)propen
1-(4-Acetoxy-3-methoxyphenyl)-2-(5,6,7,8-tetrahydro-5,5,8,8-tetramethyl-2-naphthalenyl)propen
1-(3,4-Diformyloxyphenyl)-2-(5,6,7,8-tetrahydro-5,5,8,8-tetramethyl-2-naphthalenyl)propen
1-(3,4-Diacetoxyphenyl)-2-(5,6,7,8-tetrahydro-5,5,8,8-tetramethyl-2-naphthalenyl)propen
1-(3,4-Dipropionyloxyphenyl)-2-(5,6,7,8-tetrahydro-5,5,8,8-tetramethyl-2-naphthalenyl)propen
1-(3,4-Dibutyloxyphenyl)-2-(5,6,7,8-tetrahydro-5,5,8,8-tetramethyl-2-naphthalenyl)propen
1-(3,4,5-Trihydroxyphenyl)-2-(5,6,7,8-tetrahydro-5,5,8,8-tetramethyl-2-naphthalenyl)propen
1-(3,4,5-Trimethoxyphenyl)-2-(5,6,7,8-tetrahydro-5,5,8,8-tetramethyl-2-naphthalenyl)propen
1-(3-Hydroxy-4,5-dimethoxyphenyl)-2-(5,6,7,8-tetrahydro-5,5,8,8-tetramethyl-2-naphthalenyl)propen
1-(4-Hydroxyphenyl)-2-(5,6,7,8-tetrahydro-5,5,8,8-tetramethyl-2-naphthalenyl)ethen
1-(4-Hydroxyphenyl)-2-(5,6,7,8-tetrahydro-5,5,8,8-tetramethyl-2-naphthalenyl)buten
1-(4-Hydroxyphenyl)-2-(5,6,7,8-tetrahydro-5,5,8,8-tetramethyl-2-naphthalenyl)penten
1-(4-Hydroxyphenyl)-2-(5,6,7,8-tetrahydro-5,5,8,8-tetramethyl-2-naphthalenyl)hexen
2-Cyclopropyl-1-(4-hydroxyphenyl)-2-(5,6,7,8-tetrahydro-5,5,8,8-tetramethyl-2-naphthalenyl)ethen
1-(4-Methoxyphenyl)-2-(5,6,7,8-tetrahydro-5,5,8,8-tetramethyl-2-naphthalenyl)ethen
1-(4-Methoxyphenyl)-2-(5,6,7,8-tetrahydro-5,5,8,8-tetramethyl-2-naphthalenyl)buten
1-(4-Acetoxyphenyl)-2-(5,6,7,8-tetrahydro-5,5,8,8-tetramethyl-2-naphthalenyl)ethen
1-(4-Hydroxyphenyl)-2-(5,6,7,8-tetrahydro-5,5,8,8-tetramethyl-2-naphthalenyl)propen
1-(4-Hydroxyphenyl)-2-(5,6,7,8-tetrahydro-3-methoxy-5,5,8,8-tetramethyl-2-naphthalenyl)propen
1-(4-Hydroxyphenyl)-2-(3-ethyl-5,6,7,8-tetrahydro-3-methoxy-5,5,8,8-tetramethyl-2-naphthalenyl)propen
1-(4-Hydroxyphenyl)-2-(3-fluor-5,6,7,8-tetrahydro-3-methoxy-5,5,8,8-tetramethyl-2-naphthalenyl)propen
1-(4-Methoxyphenyl)-2-(5,6,7,8-tetrahydro-5,5,8,8-pentamethyl-2-naphthalenyl)propen
1-(4-Methoxyphenyl)-2-(5,6,7,8-tetrahydro-3-methoxy-5,5,8,8-naphthalenyl)propen
1-(4-Methoxyphenyl)-2-(3-ethyl-5,6,7,8-tetrahydro-5,5,8,8-tetramethyl-2-naphthalenyl)propen
1-(4-Methoxyphenyl)-2-(3-fluor-5,6,7,8-tetrahydro-5,5,8,8-tetramethyl-2-naphthalenyl)propen
1-(4-Hydroxyphenyl)-2-(2,3-dihydro-1,1,3,3-tetramethyl-5(1H)-indenyl)-propen
1-(4-Methoxyphenyl)-2-(2,3-dihydro-1,1,3,3-tetramethyl-5(1H)-indenyl)-propen
1-(4-Acetoxyphenyl)-2-(2,3-dihydro-1,1,3,3-tetramethyl-5(1H)-indenyl)-propen
1-(4-Hydroxyphenyl)-2-(2,3-dihydro-1,1,2,3,3-pentamethyl-5(1H)-indenyl)-propen
1-(4-Methoxyphenyl)-2-(2,3-dihydro-1,1,2,3,3-pentamethyl-5(1H)-indenyl)-propen
1-(4-Acetoxyphenyl)-2-(2,3-dihydro-1,1,2,3,3-pentamethyl-5(1H)-indenyl)-propen
1-(4-Hydroxyphenyl)-2-(5,6,7,8-tetrahydro-3,8,8-trimethyl-2-naphthal-enyl)-propen
1-(4-Methoxyphenyl)-2-(5,6,7,8-tetrahydro-3,8,8-trimethyl-2-naphthal-enyl)-propen
1-(4-Acetoxyphenyl)-2-(5,6,7,8-tetrahydro-3,8,8-trimethyl-2-naphthal-enyl)-propen

Die erfindungsgemäßen Verbindungen kann man auf verschiedenen Wegen herstellen, die im Prinzip jeweils bekannt sind. z. B. kann man eine Carbonylverbindung der Formel II

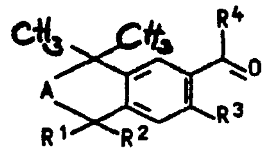

II.

in der A, $R^1$, $R^2$, $R^3$ und $R^4$ die angegebenen Bedeutungen haben, mit einer Phosphorverbindung der Formel III,

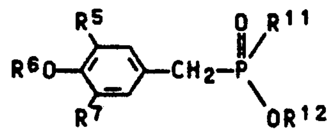

III,

in der $R^5$, $R^6$ und $R^7$ die angegebenen Bedeutungen haben und $R^{11}$ und $R^{12}$ eine $C_1$-$C_3$-Alkylgruppe bedeuten, nach Wittig-Horner umsetzen. Zweckmäßigerweise arbeitet man in einem Lösungsmittel in Gegenwart der für Wittig-Horner-Reaktionen üblichen basischen Verbindungen.

Die Umsetzung nach Wittig-Horner verläuft bei einer Temperatur von -20 bis 100°C zweckmäßigerweise bei 20 bis 50°C. Die Umsetzung kann bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzung in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylformanid oder deren Mischungen, wobei die Umsetzung im allgemeinen bei einer Temperatur von 0 bis 30°C abläuft.

Die Umsetzungen werden in Gegenwart eines Deprotonierungsmittels für das Phosphonat (III) vorgenommen. Geeignet sind Alkalimetallhydride und Alkalimetallamide, insbesondere des Natriums und Kaliums, die Natrium- oder Kaliumsalze von Dimethylsulfoxid, Alkyllithiumverbindungen wie n-Butyllithium oder Alkalimetallalkoholate, vorzugsweise Natriumethanolat und Natriummethanolat.

Man kann die erfindungsgemäßen Verbindungen auch durch Wittig-Reaktion eines Phosphoniumsalzes der Formel IV

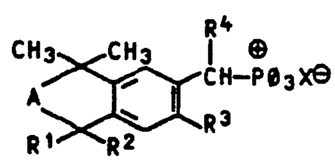

IV,

in der A, $R^1$, $R^2$, $R^3$ und $R^4$ die oben angegebene Bedeutungen haben und $X^\ominus$ für ein Anion, vorzugsweise Chlor oder Brom, steht, mit einem Benzaldehyd der Formel V

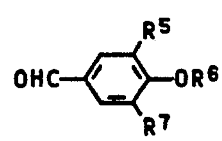

V,

wobei $R^5$, $R^6$ und $R^7$ die genannten Bedeutungen haben, erhalten.

Die Wittig- oder Wittig-Horner-Reaktion liefert üblicherweise Gemische der sterisch isomeren (E/Z)-Olefine.

E/Z-Isomerengemische mit überwiegendem Z-Anteil werden unter Lichteinwirkung an der olefinischen Doppelbindung zu Gemischen mit höherem Anteil der (E)-Isomeren umgelagert. Aus den erhaltenen (E/Z)-Isomerengemischen mit jetzt günstigerem (E)-Gehalt werden vorteilhaft reine (E)-Verbindungen der Formel (I), vorzugsweise durch Kristallisation oder eine chromatographische Methode wie die Säulen- oder präparative HPL-Chromatographie erhalten.

Carbonsäureester der allgemeinen Formel I werden, falls dies erwünscht ist, in die freien Carbonsäuren bzw. in die freien Phenole und ihre physiologisch verträglichen Salze, durch Esterverseifung übergeführt. Zweckmäßigerweise erfolgt die Verseifung in Gegenwart eines Verdünnungsmittels, beispielsweise eines mit Wasser mischbaren Ethers, wie 1,2-Di-methoxyethan oder Tetrahydrofuran, oder eines niederen aliphatischen Alkohols, wie Methanol, Ethanol, Propanol, Isopropanol oder Butanol, gegebenenfalls in Gegenwart von

Wasser bzw. in Mischungen der genannten Lösungsmittel mit Wasser. Bevorzugte Lösungsmittel sind wäßrige Mischungen von Ethanol oder Methanol, wobei die Umsetzung zwischen 20°C und dem Siedepunkt des Reaktionsgemisches durchgeführt wird. Die Verseifung geschieht bevorzugt in Gegenwart von Hydroxiden oder Carbonaten der Alkali- und Erdalkalimetalle, insbesondere des Natriums und Kaliums.

Umgekehrt kann eine Carbonsäure der Formel I in an sich bekannter Weise verestert werden. So kann man beispielsweise die Carbonsäure unter Einwirkung eines anorganischen Säurechlorids, vorzugsweise Thionylchlorid, in das entsprechende Säurechlorid überführen und dieses anschließend mit dem gewünschten Alkohol umsetzen, wobei man zweckmäßigerweise einen großen Überschuß des Alkohols einsetzt und gegebenenfalls in einem inerten Lösungsmittel, z. B. einem Kohlenwasserstoff wie Toluol arbeitet. Als vorteilhaft erweist sich in manchen Fällen auch der Zusatz einer tertiären Stickstoffbase wie Triethylamin oder Pyridin, um den gebildeten Halogenwasserstoff zu binden.

Die Veresterung geschieht auch vorteilhaft, indem man die Carbonsäure zunächst in ihr Salz überführt und dieses mit einem entsprechenden Alkylhalogenid, vorzugsweise einem Alkylbromid oder -iodid, behandelt. Als Deprotonierungsmittel für die Herstellung der Salze in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid und insbesondere Methylethylketon, wobei die Umsetzung beim Siedepunkt des Reaktionsgemisches durchgeführt wird.

Ein Phenol der Formel I kann in an sich bekannter Weise mit einem Alkanoylhalogenid oder -anhydrid, einem Aralkanoylhalogenid oder -anhydrid oder einem Aroylhalogenid oder -anhydrid zweckmäßigerweise in einem inerten Verdünnungs- oder Lösungsmittel, z. B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungsmittel oder Lösungsmittel in die erfindungsgemäßen Ester überführt werden. Bevorzugt werden die Umsetzungen in Gegenwart einer Base als säurebindendes Mittel in einem zwischen -20°C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Die Veretherung der Phenole der Formel I zu Arylethern der Formel I geschieht vorteilhaft, indem man das Phenol zunächst in sein Salz überführt und dieses mit einem entsprechenden Alkylhalogenid, oder -sulfat, vorzugsweise einem Alkylchlorid, -bromid oder -jodid, behandelt. Als Deprotonierungsmittel für die Herstellung der Phenolate in situ eignen sich insbesondere die Carbonate, Hydroxide und Hydride der Akalimetalle. Zweckmäßigerweise verwendet man aprotische Polare Lösungsmittel wie Aceton, Dimethylformamid, Dimethylsulfoxid oder Methylethylketon, wobei die Umsetzung zwischen 20°C und dem Siedepunkt des Reaktionsgemisches durchgeführt wird.

Brenzkatechinderivate der Formel I ($R^5$ = OH und $R^6$ = H) können nach der oben für die Acylierung von Phenolen beschriebenen Verfahrensweise mit Phosgen bzw. Thiophosgen zu den entsprechenden Carbonaten bzw. Thiocarbonaten umgesetzt werden.

Weiterhin können Brenzkatechinderivate der Formel I ($R^5$ = OH und $R^6$ = H) in an sich bekannter Weise mit Ketonen oder Aldehyden in entsprechende Benzodioxolderivate übergeführt werden. Zweckmäßigerweise verfährt man dabei so, daß man in Gegenwart einer Säure als Katalysator arbeitet und das bei der Reaktion entstehende Wasser durch azeotrope Destillation am Wasserabscheider entfernt. Als Säuren finden Mineralsäuren wie Salzsäure und Schwefelsäure, oder organische Säuren, insbesondere Sulfonsäuren wie z. B. Benzol- oder Toluolsulfonsäure Verwendung. Als Wasserschlepper werden, wie bei diesem Reaktionstyp üblich, Lösungsmittel wie Toluol, Benzol, gesättigte Kohlenwasserstoffe, wie z. B. Petrolether, oder chlorierte Kohlenwasserstoffe, wie z. B. Chloroform oder Tetrachlorkohlenstoff eingesetzt. Vorteilhaft ist ein Überschuß des Ketons oder Aldehyds.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium-, Alkalimetallsalze, insbesondere des Natriums, Kaliums und Lithiums, Erdalkalimetallsalze, insbesondere des Calciums oder Magnesiums, sowie Salze mit geeigneten organischen Basen, wie mit niederen Alkylaminen, z. B. Methylamin, Ethylamin oder Cyclohexylamin, oder mit subtituierten Alkylaminen, wie Diethanolamin, Triethanolamin oder Tris-(hydroxymethyl)-aminomethan, sowie mit Piperidin oder Morpholin.

Gegebenenfalls werden die erhaltenen erfindungsgemäßen Amine der Formel (I) nach bekannter Verfahrensweise in das Säureadditionssalz einer physiologisch verträglichen Säure überführt. Als übliche physiologisch verträgliche organische oder anorganische Säuren kommen beispielsweise in Betracht: Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure. Weitere Beispiele können aus Fortschritte der Arzneimittelforschung, Band 10, Seiten 224 bis 225, Birkhäuser Verlag, Basel und Stuttgart, 1966, entnommen werden.

Die erfindungsgemäßen Verbindungen und ihre physiologisch verträglichen Salze können aufgrund ihrer pharmakologischen Eigenschaften bei der topischen und systemischen Therapie und auch Prophylaxe von

praekanzerosen und Karzinomen der Haut, der Schleimhäute und innerer Organe sowie bei der topischen und systemischen Therapie von Akne, Psoriasis und anderer mit pathologisch veränderter Verhornung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungenn die prophylaktische und therapeutische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res., 36, 964 bis 972 (1976), aus Nature 250, 64 bis 66 (1974), aus Nature 253, 47 bis 50 (1975) und aus Cancer Res., 43, 24695 (1983) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035 bis 1041 (1978), Experimental Cell Research 117, 15 bis 22 (1978) und proc. Natl. Acad. Sci., USA 77, 2937 bis 2940 (1980) entnommen werden.

Die antiarthritische Wirkung der erfindungsgemäßen Verbindungen kann in üblicher Weise im Tierexperiment im Adjuvans-Arthritis-Modell oder im Streptokokkenzellwandinduzierte-Arthritis-Modell bestimmt werden. Diese Methodik kann z. B. aus "Retinoids, differentiation and disease", Ciba Foundation Symposium 113, S. 191 bis 211, Pitman, London, 1985, entnommen werden.

Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al in The Journal of Investigative Dermatology 73, 354 bis 358 (1978 beschrieben.

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1 %-iger Konzentration, bevorzugt in 0,001 bis 0,1 %-iger Konzentration und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleichmittel usw. zugesetzt werden. Voraussetzung ist, daß alle bei der Herstellung pharmazeutischer Zubereitungen verwendeten Stoffe toxikologisch unbedenklich sind und mit den verwendeten Wirkstoffen verträglich sind.

**Beispiel 1**

Methode A:

(E)-1-(4-Methoxyphenyl)-2-(5,6,7,8-tetrahydro-5,5,8,8-tetramethyl-2-naphthalenyl)propen

Zu einer Suspension von 6 g (0,2 mol) Natriumhydrid (80 %-ig, zuvor mit Petrolether vom 20 %-igen Paraffinanteil befreit) in 200 ml absolutem Tetrahydrofuran wurde bei 40 bis 45° C eine Lösung von 100,4 g (0,18 mol) 1-(5,6,7,8-Tetrahydro-5,5,8,8-tetramethyl-2-naphthalenyl)-ethyl-tri-phenylphosphoniumbromid in 300 ml absolutem Dimethylsulfoxid zugetropft. Man ließ 0,5 h bis zum Ende der Gasentwicklung nachrühren, gab nochmals 50 ml Tetrahydrofuran zu und tropfte bei ca. 30° C innerhalb 20 Min. eine Lösung von 21,7 g (0,16 mol) 4-Methoxybenzaldehyd in 80 ml Tetrahydrofuran zu. Man ließ über Nacht nachrühren, goß danach auf 1 l

Wasser und extrahierte dreimal mit Ether. Die vereinigten Etherphasen wurden einmal mit Wasser gewaschen, über $Na_2SO_4$ getrocknet und eingeengt. Der semikristalline Rückstand (102,0 g) wurde in 600 ml n-Heptan bis zum Siedepunkt erhitzt und wieder abkühlen gelassen. Vom Feststoff (hauptsächlich Triphenylphosphinoxid) wurde abfiltriert und das Filtrat eingeengt. Der Rückstand wurde in 550 ml Methanol umkristallisiert. Nach Trocknen erhielt man 26,4 g der Titelverbindung, Schmp. 103 bis 104°C.

Methode B:

Zu einer Lösung von 26,3 g (0,1 mol) p-Methoxybenzylphosphonsäurediethylester in 400 ml abs. Dimethylformamid wurden unter Stickstoff und bei Raumtemperatur 11,5 g (0,1 mol) Kalium-tert.-butanolat portionsweise zugegeben. Man ließ 30 Min. nachrühren und tropfte dann eine Lösung von 11,5 g (0 05 mol) 2-Acetyl-5,6,7,8-tetrahydro-5,5,8,8-tetramethyl-naphthalin in 100 ml abs. Dimethylformamid bei Raumtemperatur zu. Anschließend rührte man 4 h bei 60°C und nochmals 4 h bei 80°C. Nach dem Abkühlen goß man auf 1 l Eiswasser und extrahierte dreimal mit je 80 ml Ether. Zur Erleichterung der Extraktion wurde angesäuert. Die vereinigten Etherextrakte wurden fünfmal mit je 200 ml $H_2O$ gewaschen, getrocknet ($MgSO_4$) und eingeengt. Der Rückstand wurde mit Methanol verrührt und bei -20°C (Tiefkühlfach) stehengelassen. Anderntags wurden die Kristalle abgesaugt. Nach Trocknen wurden so 6 g der Titelverbindung erhalten, nach physikalischen Daten identisch mit der nach Methode A hergestellten Verbindung.

**Beispiel 2**

(E)-1-(4-Butoxyphenyl)-2-(5,6,7,8-tetrahydro-5,5,8,8-tetramethyl-2-naphthalenyl)propen

Analog Beispiel 1 wurden aus 6 g (0,2 mol) Natriumhydrid (80 %-ig), 100,4 g (0,18 mol) 1-(5,6,7,8-Tetrahydro-5,5,8,8-tetramethyl-2-naphthalenyl)ethyl-triphenylphosphoniumbromid und 28,5 g (0,16 mol) 4-tert.-Butoxybenzaldehyd 11,5 g der Titelverbindung erhalten, Schmp. 107 bis 108°C. Das nach der Heptanextraktion verbliebene Rohkristallisat wurde mittels Blitzchromatographie (150 g Kieselgel 60, 60 bis 230 mesh; n-Heptan) vorgereinigt und schließlich aus Tetrahydrofuran/Ethanol/Methanol (2 : 1 : 8) umkristallisiert.

**Beispiel 3**

(E)-5-[2-(5,6,7,8-Tetrahydro-5,5,8,8-tetramethyl-2-naphthalenyl)-1-propenyl]-1,3-benzodioxol

Analog Beispiel 1 wurden aus 1,5 g (0,05 mol) Natriumhydrid (80 %-ig), 25,1 g (0,045 mol) 1-(5,6,7,8-Tetrahydro-5,5,8,8-tetramethyl-2-naphthalenyl)ethyl-triphenylphosphoniumbromid und 6,0 g (0,04 mol) Piperonal 4,3 g der Titelverbindung erhalten, Schmp. 90 bis 91°C.

**Beispiel 4**

(E)-4-[2-(5,6,7,8-tetrahydro-5,5,8,8-tetramethyl-2-naphthalenyl)-1-propenyl]-phenoxyessigsäureethylester

Analog Beispiel 1 wurden aus 1,5 g (0,05 mol) Natriumhydrid (80 %-ig), 25,1 g (0,045 mol) 1-(5,6,7,8-Tetrahydro-5,5,8,8-tetramethyl-2-naphthalenyl)ethyl-triphenylphosphoniumbromid und 8,3 g (0,04 mol) 4-Formylphenoxyessigsäureethylester 2,5 g der Titelverbindung erhalten, Schmp. 71 bis 73°C. Das nach der Heptanextraktion verbliebene ölige Rohprodukt wurde mittels Blitzchromatographie (200 g Kieselgel 60, 230 bis 400 mesh: n-Heptan + 1 % Essigester) vorgereinigt und schließlich aus Methanol/Ethanol (1 : 3) umkristallisiert.

**Beispiel 5**

(E)-1-(3,4-Diacetoxyphenyl)-2-(5,6,7,8-tetrahydro-5,5,8,8-tetramethyl-2-naphthalenyl)propen

Analog Beispiel 1 wurden aus 3,0 g (0,1 mol) Natriumhydrid (80 %-ig), 50,4 g (0,09 mol) 1-(5,6,7,8-Tetrahydro-5,5,8,8-tetramethyl-2-naphthalenyl)ethyl-triphenylphosphoniumbromid und 15,2 g (0,08 mol) 3,4-Diacetoxybenzaldehyd 7,0 g der Titelverbindung erhalten, Schmp. 135 bis 137°C.

**Beispiel 6**

(E)-1-(4-Hydroxyphenyl)-2-(5,6,7,8-tetrahydro-5,5,8.8-tetramethyl-2-naphthalenyl)propen

3,8 g (11 mmol) (E)-1-(4-Methoxyphenyl)-2-(5,6,7,8-tetrahydro-5,5,8,8-tetramethyl-2-naphthalenyl)propen aus Beispiel 1 wurden in 50 ml mit Chlorwasserstoff gesättigtem Methanol suspendiert und 20 Min. bei 60°C

gerührt. Anschließend goß man die Reaktionslösung in ca. 150 ml Eiswasser und extrahierte dreimal mit je 100 ml Methyl-tert.-butylether. Die organische Phase wurde zweimal mit je 100 ml gesättigter Natriumchloridlösung und zweimal mit Wasser gewaschen. Nach Trocknen über $Na_2SO_4$ und Einengen blieben 4 g festes Rohprodukt zurück. Daraus erhielt man nach Umkristallisation aus Isopropanol 1,5 g der Titelverbindung, Schmp. 138 bis 140°C.

**Beispiel 7**

(E)-4-[2-(5,6,7,8-tetrahydro-5,5,8,8-tetramethyl-2-naphthalenyl)-1-propenyl]phenoxyessigsäure

1,6 g (4 mmol) (E)-4-[2-(5,6,7,8-tetrahydro-5,5,8,8-tetramethyl-2-naphthalenyl)-1-propenyl]phenoxy essigsäureethylester aus Beispiel 4 und 0,5 g (8 mmol) Kaliumhydroxid (85 %ig) wurden in einem Gemisch aus 20 ml Ethanol und 1 ml Wasser 1 h bei Rückfluß erhitzt. Danach ließ man abkühlen, goß auf 100 ml Wasser und säuerte mit verdünnter HCL an. Nach einiger Zeit bildete sich ein Kristallisat, das abgesaugt und mit Wasser und wenig eiskaltem Methanol gewaschen wurde. Nach dem Trocknen erhielt man 1,2 g der Titelverbindung, Schmp. 152 bis 154°C.

**Patentansprüche** für die Vetragsstaaten: BE, CH, DE, ES, FR, GB, GR, IT, LT, NL, SE,

1. Vinylphenolderivate der Formel I

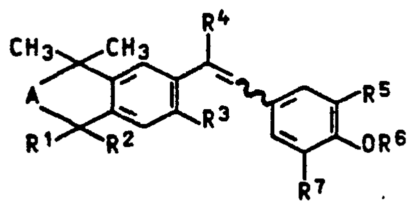

in der

A einen gegebenenfalls mit $C_1$-$C_4$-Alkylgruppen substituierten Methylen- oder Ethylenrest,
$R^1$ und $R^2$ Wasserstoffatome oder Methylgruppen,
$R^3$ ein Wasserstoffatom, eine $C_1$-$C_4$-Alkylgruppe, eine $C_1$-$C_4$-Alkoxygruppe oder ein Halogenatom,
$R^4$ ein Wasserstoffatom, eine $C_1$-$C_4$-Alkylgruppe, Cyclopropyl oder Cyclobutyl,
$R^5$ und $R^7$ je ein Wasserstoffatom oder die Gruppe -$OR^8$, worin $R^8$ für ein Wasserstoffatom, eine $C_1$-$C_4$-Alkylgruppe oder eine $C_1$-$C_4$-Alkanoylgruppe steht, und
$R^6$ ein Wasserstoffatom, eine gegebenenfalls mit Carboxyl-, $C_1$-$C_4$-Alkoxycarbonyl, Hydroxyl-, $C_1$-$C_4$-Alkoxy- und/oder Amino-, Mono- oder Di-$C_1$-$C_4$-Alkylaminogruppen substituierte $C_1$-$C_4$-Alkylgruppe, eine $C_1$-$C_{20}$-Alkanoylgruppe, eine gegebenenfalls wie für die $C_1$-$C_4$-Alkylgruppe beschrieben substituierte Benzoyl- oder eine gegebenenfalls im Arylteil ebenso substituierte Aralkylgruppe darstellen, wobei $R^6$ und $R^8$ auch gemeinsam für einen Rest C=O, C=S, -$CH_2CH_2$- oder $CR^9R^{10}$ stehen können, mit $R^9$ und $R^{10}$ in der Bedeutung eines Wasserstoffatoms, einer $C_1$-$C_4$-Alkylgruppe oder einer Phenylgruppe,
sowie deren physiologisch verträglichen Salze.

2. (E)-1-(4-Methoxyphenyl)-2-(5,6,7,8-tetrahydro-5,5,8,8-tetramethyl-2-naphthalenyl)propen.

3. (E)-1-(4-Hydroxyphenyl)-2-(5,6,7,8-tetrahydro-5,5,8,8-tetramethyl-2-naphthalenyl)propen.

4. Vinylphenolderivate nach einem der Ansprüche 1 bis 3 als Heilmittel.

5. Arzneimittel zur topischen oder systemischen Bekämpfung von Akne, Psoriasis und anderen mit pathologisch veränderter Verhornung einhergehenden dermatologischen Erkrankungen, von Praekanzerosen und Karzinomen der Haut, der Schleimhäute und innerer Organe sowie zur Behandlung von rheumatischen Erkrankungen, das neben üblichen pharmazeutisch-technischen Hilfsstoffen eine wirksame Menge eines Vinylphenolderivates nach einem der Ansprüche 1 bis 3 als Wirkstoff enthält.

6. Verwendung eines Vinylphenolderivates nach einem der Ansprüche 1 bis 3 zur Herstellung eines Arzneimittels zur topischen oder systemischen Bekämpfung von Akne, Psoriasis und anderen mit pathologisch veränderter Verhornung einhergehenden dermatologischen Erkrankungen, von Praekanzerosen und Karzinomen der Haut, der Schleimhäute und innerer Organe sowie zur Behandlung von rheumatischen Erkrankungen.

**Patentansprüche** für den Vertragsstaat Österreich

1. Verfahren zur Herstellung von Vinylphenolderivaten der Formel I

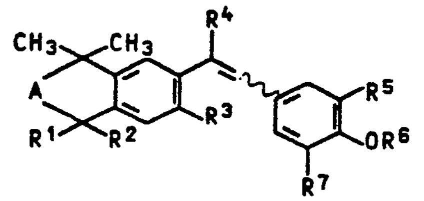

in der
A einen gegebenenfalls mit $C_1$-$C_4$-Alkylgruppen substituierten Methylen- oder Ethylenrest,
$R^1$ und $R^2$ Wasserstoffatome oder Methylgruppen,
$R^3$ ein Wasserstoffatom, eine $C_1$-$C_4$-Alkylgruppe, eine $C_1$-$C_4$-Alkoxygruppe oder ein Halogenatom,
$R^4$ ein Wasserstoffatom, eine $C_1$-$C_4$-Alkylgruppe, Cyclopropyl oder Cyclobutyl,
$R^5$ und $R^7$ je ein Wasserstoffatom oder die Gruppe $-OR^8$, worin $R^8$ für ein Wasserstoffatom, eine $C_1$-$C_4$-Alkylgruppe oder eine $C_1$-$C_4$-Alkanoylgruppe steht, und
$R^6$ ein Wasserstoffatom, eine gegebenenfalls mit Carboxyl-, $C_1$-$C_4$-Alkoxycarbonyl, Hydroxyl-, $C_1$-$C_4$-Alkoxy- und/oder Amino-, Mono- oder Di-$C_1$-$C_4$-Alkylaminogruppen substituierte $C_1$-$C_4$-Alkylgruppe, eine $C_1$-$C_{20}$-Alkanoylgruppe, eine gegebenenfalls wie für die $C_1$-$C_4$-Alkylgruppe beschrieben substituierte Benzoyl- oder eine gegebenenfalls im Arylteil ebenso substituierte Aralkylgruppe darstellen, wobei $R^6$ und $R^8$ auch gemeinsam für einen Rest C=O, C=S, $-CH_2CH_2-$ oder $CR^9R^{10}$ stehen können, mit $R^9$ und $R^{10}$ in der Bedeutung eines Wasserstoffatoms, einer $C_1$-$C_4$-Alkylgruppe oder einer Phenylgruppe,
sowie deren physiologisch verträglichen Salzen,
a) indem man eine Carbonylverbindung der Formel II

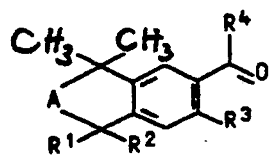
II,

in der A, $R^1$, $R^2$, $R^3$ und $R^4$ die angegebenen Bedeutungen haben, mit einer Phosphorverbindung der Formel III,

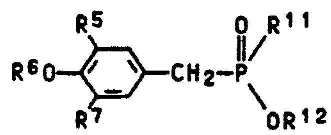
III,

in der $R^5$, $R^6$ und $R^7$ die angegebenen Bedeutungen haben und $R^{11}$ und $R^{12}$ eine $C_1$-$C_3$-Alkylgruppe bedeuten, nach Wittig-Horner bei -20 bis 100° C umsetzt,
oder
b) durch Wittig-Reaktion eines Phosphoniumsalzes der Formel IV

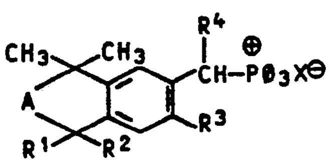
IV,

in der A, $R^1$, $R^2$, $R^3$ und $R^4$ die oben angegebene Bedeutungen haben und $X^{\ominus}$ für ein Anion, vorzugsweise Chlor oder Brom, steht, mit einem Benzaldehyd der Formel V

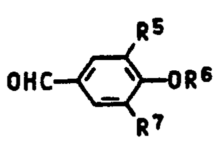
V,

wobei $R^5$, $R^6$ und $R^7$ die genannten Bedeutungen haben,
und gegebenenfalls anschließende Umsetzung in üblicher Weise zu physiologisch verträglichen Salzen.

2. Arzneimittel zur topischen oder systemischen Bekämpfung von Akne, Psoriasis und anderen mit pathologisch veränderter Verhornung einhergehenden dermatologischen Erkrankungen, von Praekanzerosen

und Karzinomen der Haut, der Schleimhäute und innerer Organe sowie zur Behandlung von rheumatischen Erkrankungen, das neben üblichen pharmazeutisch-technischen Hilfsstoffen eine wirksame Menge eines nach einem Verfahren des Anspruchs 1 erhältlichen Vinylphenolderivates als Wirkstoff enthält.

**Claims** for the Contracting States: BE, CH, DE, ES, FR, GB, GR, IT, LI, NL, SE

1. A vinylphenol derivative of the formula I

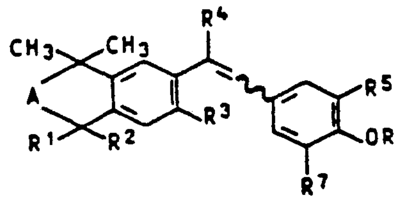

where
A is a methylene or ethylene radical which is unsubstituted or substituted by $C_1$-$C_4$-alkyl,
$R^1$ and $R^2$ are each hydrogen or methyl,
$R^3$ is hydrogen, $C_1$-$C_4$-alkyl, $C_1$-$C_4$-alkoxy or halogen,
$R^4$ is hydrogen, $C_1$-$C_4$-alkyl, cyclopropyl or cyclobutyl,
$R^5$ and $R^7$ are each hydrogen or $-OR^8$, where $R^8$ is hydrogen, $C_1$-$C_4$-alkyl or $C_1$-$C_4$-alkanoyl, and
$R^6$ is hydrogen or is $C_1$-$C_4$-alkyl which is unsubstituted or substituted by carboxyl, $C_1$-$C_4$-alkoxycarbonyl, hydroxyl, $C_1$-$C_4$-alkoxy and/or amino or mono- or di-$C_1$-$C_4$-alkylamino, or is $C_1$-$C_{20}$-alkanoyl or is benzoyl which is unsubstituted or substituted as described for the $C_1$-$C_4$-alkyl group, or is aralkyl which is unsubstituted or similarly substituted in the aryl moiety, and $R^6$ and $R^8$ together may furthermore form a radical C=O, C=S, $-CH_2CH_2-$ or $CR^9R^{10}$, where $R^9$ and $R^{10}$ are each hydrogen, $C_1$-$C_4$-alkyl or phenyl,
and its physiologically tolerated salts.

2. (E)-1-(4-methoxyphenyl)-2-(5,6,7,8-tetrahydro-5,5,8,8-tetramethylnaphth-2-yl)-propene.
3. (E)-1-(4-hydroxyphenyl)-2-(5,6,7,8-tetrahydro-5,5,8,8-tetramethylnaphth-2-yl)-propene.
4. A vinylphenol derivative as claimed in any of claims 1 to 3, as a drug.
5. A drug for topical or systemic control of acne, psoriasis and other dermatological disorders accompanied by pathologically changed keratinization, and of precancers and carcinomas of the skin, the mucous membranes and the internal organs and for the treatment of rheumatic disorders, which contains, in addition to conventional pharmaceutical auxiliaries, an effective amount of a vinylphenol derivative as claimed in any of claims 1 to 3, as active compound.
6. Use of a vinylphenol derivative as claimed in any of claims 1 to 3 for the preparation of a drug for the topical or systemic control of acne, psoriasis and other dermatological disorders accompanied by pathologically changed keratinization, and of precancers and carcinomas of the skin, the mucous membranes and the internal organs, and for the treatment of rheumatic disorders.

**Claims** for the Contracting State: AT

1. A process for the preparation of a vinylphenol derivative of the formula I

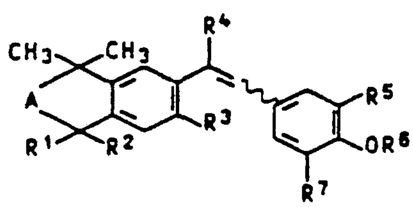

where
A is a methylene or ethylene radical which is unsubstituted or substituted by $C_1$-$C_4$-alkyl,
$R^1$ and $R^2$ are each hydrogen or methyl,
$R^3$ is hydrogen, $C_1$-$C_4$-alkyl, $C_1$-$C_4$-alkoxy or halogen,
$R^4$ is hydrogen, $C_1$-$C_4$-alkyl, cyclopropyl or cyclobutyl,
$R^5$ and $R^7$ are each hydrogen or $-OR^8$, where $R^8$ is hydrogen, $C_1$-$C_4$-alkyl or $C_1$-$C_4$-alkanoyl, and

$R^6$ is hydrogen or is $C_1$-$C_4$-alkyl which is unsubstituted or substituted by carboxyl, $C_1$-$C_4$-alkoxycarbonyl, hydroxyl, $C_1$-$C_4$-alkoxy and/or amino or mono- or di-$C_1$-$C_4$-alkylamino, or is $C_1$-$C_{20}$-alkanoyl or is benzoyl which is unsubstituted or substituted as described for the $C_1$-$C_4$-alkyl group, or is aralkyl which is unsubstituted or similarly substituted in the aryl moiety, and $R^6$ and $R^8$ together may furthermore form a radical C=O, C=S, $-CH_2CH_2-$ or $CR^9R^{10}$, where $R^9$ and $R^{10}$ are each hydrogen, $C_1$-$C_4$-alkyl or phenyl,

and its physiologically tolerated salts,

a) wherein a carbonyl compound of the formula II

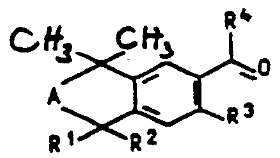

II.

where A, $R^1$, $R^2$, $R^3$, $R^4$ have the stated meanings is subjected to a Wittig-Horner reaction, at from -20 to 100°C with a phosphorus compound of the formula III

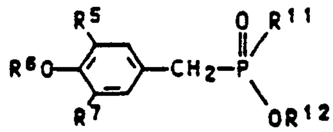

III.

where $R^5$, $R^6$ and $R^7$ have the stated meanings and $R^{11}$ and $R^{12}$ are each $C_1$-$C_3$-alkyl, or

b) a phosphonium salt of the formula IV

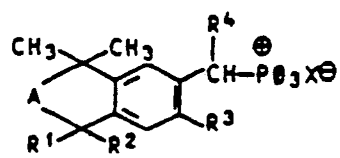

IV.

where A, $R^1$, $R^2$, $R^3$ and $R^4$ have the above meanings and X is an anion, preferably chlorine or bromine, is subjected to a Wittig reaction with a benzaldehyde of the formula V

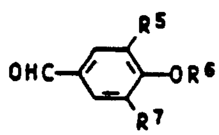

V.

where $R^5$, $R^6$ and $R^7$ have the stated meanings, and, if required, the product is then converted in a conventional manner to a physiologically tolerated salt.

2. A drug for topical or systemic control of acne, psoriasis and other dermatological disorders accompanied by pathologically changed keratinization, and of precancers and carcinomas of the skin, the mucous membranes and the internal organs and for the treatment of rheumatic disorders, which contains, in addition to conventional pharmaceutical auxiliaries, an effective amount of a vinylphenol derivative obtainable by a process of claim 1, as active compound.

**Revendications** pour les Etats contractants: BE, CH, DE, ES, FR, GB, GR, IT, LI, NL, SE

1. Dérivés du vinylphénol répondant à la formule I

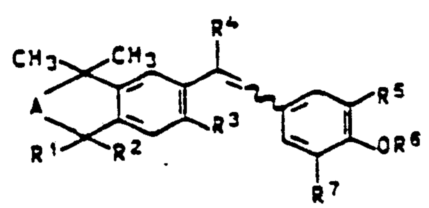

I

dans laquelle

A représente un groupe méthylène ou éthylène éventuellement substitué par des groupes alkyle en C 1-C 4,

$R^1$ et $R^2$ représentent des atomes d'hydrogène ou des groupes méthyle,

$R^3$ represente un atome d'hydrogène, un groupe alkyle en C 1-C 4, un groupe alcoxy en C 1-C 4 ou un atome d'halogène,

$R^4$ représente un atome d'hydrogène, un groupe alkyle en C 1-C 4, cyclopropyle ou cyclobutyle,

$R^5$ et $R^7$ représentent chacun un atome d'hydrogène ou le groupe $-OR^8$ dans lequel $R^8$ représente un atome d'hydrogène, un groupe alkyle en C 1-C 4 ou un groupe alcanoyle en C 1-C 4, et

$R^6$ represente un atome d'hydrogène, un groupe alkyle en C 1-C4 éventuellement substitué par des groupes carboxyle, (alcoxy en C1-C 4)-carbonyle, hydroxy, alcoxy en C 1-C 4 et/ou amino, mono- ou di-(alkyle en C 1-C 4)-amino, un groupe alcanoyle en C 1-C 20, un groupe benzoyle éventuellement substitué comme indiqué ci-dessus pour le groupe alkyle en C 1-C 4 ou un groupe aralkyle portant éventuellement des substituants du même type dans la partie aryle, $R^6$ et $R^8$ pouvant également former ensemble un groupe C=O, C=S,$-CH_2CH_2-$ ou $CR^9$ $R^{10}$ dans lequel $R^9$ et $R^{10}$ représentent chacun un atome d'hydrogène, un groupe alkyle en C 1-C 4 ou un groupe phényle,

et leurs sels acceptables pour l'usage pharmaceutique.

2. Le (E)-1-(4-méthoxyphényl)-2-(5,6,7,8-tétrahydro-5,5,8,8-tétraméthyl-2-naphtalényl)-propène.

3. Le (E)-1-(4-hydroxyphényl)-2-(5,6,7,8-tétrahydro-5,5,8,8-tétraméthyl-2-naphtalényl)-propène.

4. Dérivés du vinylphénol selon l'une des revendications 1 à 3 en tant que médicaments.

5. Composition thérapeutique pour le traitement topique ou systématique de l'acné, du psoriasis et d'autres maladies dermatologiques provoquant une kératinisation à caractère pathologique, des précancéroses et des carcinomes de la peau, des muqueuses et des organes internes, ainsi que pour le traitement des maladies rhumatismales, qui contient, avec des produits auxiliaires pharmaceutiques usuels, une quantité efficace d'un dérivé de vinylphénol selon l'une des revendications 1 à 3 en tant que substance active.

6. Utilisation d'un dérivé de vinylphénol selon l'une des revendications 1 à 3 pour la préparation d'une composition thérapeutique pour le traitement topique ou systématique de l'acné, du psoriasis et d'autre maladies dermatologiques provoquant une kératinisation à caractère pathologique, des précancéroses et des carcinomes de la peau, des muqueuses et des organes internes, ainsi que pour le traitement des maladies rhumatismales.

**Revendications** pour l'Etat contractant: AT

1. Procédé de préparation des dérivés de vinylphénol de formule I

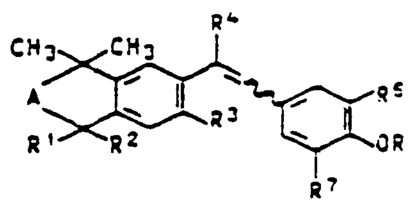

I.

dans laquelle

A représente un groupe méthylène ou éthylène éventuellement substitué par des groupes alkyle en C 1-C 4,

$R^1$ et $R^2$ représentent des atomes d'hydrogène ou des groupes méthyle,

$R^3$ représente un atome d'hydrogène, un groupe alkyle en C 1-C 4, un groupe alkoxy en C 1-C 4 ou un atome d'halogène,

$R^4$ représente un atome d'hydrogène, un groupe alkyle en C 1-C 4, cyclopropyle ou cyclobutyle,

$R^5$ et $R^7$ représentent chacun un atome d'hydrogène ou le groupe $-OR^8$ dans lequel $R^8$ représente un atome d'hydrogène, un groupe alkyle en C 1-C 4 ou un groupe alcanoyle en C 1-C 4, et

$R^6$ représente un atome d'hydrogène, un groupe alkyle en C 1-C 4 éventuellement substitué par des groupes carboxyle, (alcoxy en C 1-C 4)- carbonyle, hydroxy, alcoxy en C 1-C 4 et/ou amino, mono- ou di-(alkyle en C 1-C 4)- amino, un groupe alcanoyle en C 1-C 20, un goupe benzoyle éventuellement substitué comme indiqué ci-dessus pour le groupe alkyle en C 1-C 4 ou un groupe aralkyle portant éventuellement dans la partie aryle des substituants identiques à ceux mentionnés ci-dessus, $R^6$ et $R^8$ pouvant également former ensemble un groupe C=O, C=S, $-CH_2CH_2-$ ou $CR^9R^{10}$ dans lequel $R^9$ et $R^{10}$ représentent chacun un atome d'hydrogène, un groupe alkyle en C 1-C 4 ou un groupe phényle,

et de leurs sels acceptables pour l'usage pharmaceutique, selon lequel

a) on fait réagir selon Witting-Horner à des températures de -20 à 100°C un composé carbonylé de formule II

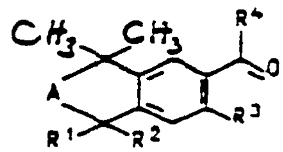

II.

dans laquelle A, $R^1$, $R^2$, $R^3$ et $R^4$ ont les significations indiquées ci-dessus, avec un composé du phosphore de formule III

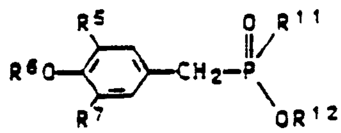

III.

dans laquelle $R^5$, $R^6$ et $R^7$ ont les significations indiquées ci-dessus et $R^{11}$ et $R^{12}$ représentent chacun un groupe alkyle en C 1-C 3, ou bien

b) on fait réagir selon Wittig un sel de phosphorium de formule IV

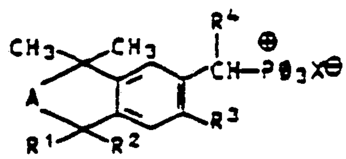

IV.

dans laquelle A, $R^1$, $R^2$, $R^3$ et $R^4$ ont les significations indiquées ci-dessus et X représente un anion, de préférence le chlore ou le brome, avec un benzaldéhyde de formule V

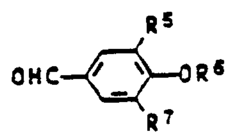

V.

dans laquelle $R^5$, $R^6$ et $R^7$ ont les significations indiquées ci-dessus et le cas échéant on convertit ensuite de la manière habituelle en sels acceptables pour l'usage pharmaceutique.

2. Composition thérapeutique pour le traitement topique ou systématique de l'acné, du psoriasis et d'autres maladies dermatologiques provoquant une kératinisation à caractère pathologique, des précancéroses et des carcimones de la peau, des muqueuses et des organes internes, ainsi que pour le traitement des maladies rhumatismales, qui contient en tant que substance active, avec des produits auxiliaires pharmaceutiques usuels, une quantité efficace d'un dérivé de vinylphénol obtenu par un procédé selon la revendication 1.